(19) **Europäisches Patentamt / European Patent Office / Office européen des brevets**

(11) **EP 3 896 120 B1**

(12) # EUROPEAN PATENT SPECIFICATION

(45) Date of publication and mention
of the grant of the patent:
**26.06.2024 Bulletin 2024/26**

(21) Application number: **19896122.9**

(22) Date of filing: **12.12.2019**

(51) International Patent Classification (IPC):
**A61L 15/24** (2006.01)     **A61L 15/60** (2006.01)
**C08J 3/075** (2006.01)     **A61F 13/53** (2006.01)
**B01J 20/26** (2006.01)     **B01J 20/28** (2006.01)
**C08F 2/32** (2006.01)     **C08J 3/12** (2006.01)

(52) Cooperative Patent Classification (CPC):
(C-Sets available)
**A61L 15/60; A61F 13/53; A61L 15/24;
B01J 20/261; B01J 20/267; B01J 20/28004;
C08F 2/32; C08J 3/075; C08J 3/12;**
A61F 2013/530481; B01J 2220/68; C08J 2333/02

(Cont.)

(86) International application number:
**PCT/JP2019/048821**

(87) International publication number:
**WO 2020/122218 (18.06.2020 Gazette 2020/25)**

(54) **WATER-ABSORBING RESIN PARTICLES, ABSORBENT, AND ABSORBENT ARTICLE**

WASSERABSORBIERENDE HARZTEILCHEN, ABSORBIERENDES MITTEL UND
ABSORBIERENDER ARTIKEL

PARTICULES DE RÉSINE ABSORBANTES, CORPS ABSORBANT, ET ARTICLE ABSORBANT

(72) Inventor: **OKAZAWA, Shiho**
**Himeji-shi, Hyogo 672-8076 (JP)**

(74) Representative: **Nederlandsch Octrooibureau**
**P.O. Box 29720**
**2502 LS The Hague (NL)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **12.12.2018   JP 2018232724
12.12.2018   JP 2018232843
12.12.2018   JP 2018232847
12.12.2018   JP 2018232726
12.12.2018   JP 2018232728
12.12.2018   JP 2018232848
12.12.2018   JP 2018232850
12.12.2018   JP 2018232851
12.12.2018   JP 2018232856
12.12.2018   JP 2018232857
30.01.2019   JP 2019014532
22.03.2019   JP 2019055308**

(43) Date of publication of application:
**20.10.2021   Bulletin 2021/42**

(73) Proprietor: **Sumitomo Seika Chemicals Co., Ltd.
Kako-gun, Hyogo 675-0145 (JP)**

(56) References cited:
**EP-A1- 1 072 630          EP-A2- 0 761 241
WO-A1-2005/027986     WO-A1-2006/014031
WO-A1-2012/053121     WO-A1-2016/158976
WO-A1-2017/170605     WO-A1-2018/062539
WO-A1-2018/155591     JP-A- H0 853 550
JP-A- H09 323 038        JP-A- 2003 088 552
JP-A- 2017 101 242**

• **DATABASE WPI Week 201208 12 November 2012
(2012-11-12) Thomson Scientific, London, GB;
AN 2012-A57639 XP002807564, & JP 2012 007062
A (SANDAIYA POLYMER KK) 12 January 2012
(2012-01-12) & JP 2012 007062 A (SANDAIYA
POLYMER KK) 12 January 2012 (2012-01-12)**

**(Cont. next page)**

- **DATABASE WPI Week 199921 16 March 1999 (1999-03-16) Thomson Scientific, London, GB; AN 1999-248515 XP002807565, & JP H11 71425 A (NIPPON SHOKUBAI CO LTD) 16 March 1999 (1999-03-16) & JP H11 71425 A (NIPPON SHOKUBAI CO LTD) 16 March 1999 (1999-03-16)**
- **DATABASE WPI Week 199923 26 March 1999 (1999-03-26) Thomson Scientific, London, GB; AN 1999-267056 XP002807566, & JP H11 80248 A (NIPPON SHOKUBAI CO LTD) 26 March 1999 (1999-03-26) & JP H11 80248 A (NIPPON SHOKUBAI CO LTD) 26 March 1999 (1999-03-26)**

(52) Cooperative Patent Classification (CPC): (Cont.)

C-Sets
**A61L 15/24, C08L 33/02**

## Description

### Technical Field

The present invention relates to water-absorbent resin particles, an absorber, and an absorbent article as set out in the appended set of claims.

### Background Art

In the related art, an absorber containing water-absorbent resin particles has been used in an absorbent article for absorbing a liquid (for example, urine) having water as a main component. For example, Patent Literature 1 below discloses water-absorbent resin particles having a particle diameter that is suitably used for absorbent articles such as diapers. In addition, Patent Literature 2 discloses a method of using a hydrogel-absorbent polymer having specific saline flow inducibility, performance under pressure, and the like as an effective absorbent member for storing a body fluid such as urine. In addition, Patent Literature 3 discloses a water absorbent agent having both a high water absorption ratio and a high water absorption rate.

### Citation List

### Patent Literature

[Patent Literature 1] Japanese Unexamined Patent Publication No. H6-345819
[Patent Literature 2] Japanese Unexamined Patent Publication No. H9-510889
[Patent Literature 3] WO2017/170605A1

### Summary of Invention

### Technical Problem

When an absorbent article absorbs a liquid, if the liquid does not diffuse sufficiently and stays only in the vicinity of the liquid absorbing portion (the portion where the liquid infiltrates), there may occur a problem that the excess liquid leaks to the outside of the absorbent article, for example, the excess liquid flows on the surface of the absorbent article. Therefore, for an absorbent article, it is required that the liquid diffuses suitably when the liquid is absorbed, and in particular, it is required that the liquid diffuses suitably in a plane direction (a direction perpendicular to a thickness direction) of the absorber when the liquid is absorbed.

An object of an aspect of the present invention is to provide water-absorbent resin particles and an absorber that provide an absorbent article in which a liquid suitably diffuses in a plane direction of the absorber when the liquid is absorbed. In addition, an object of another aspect of the present invention is to provide an absorbent article in which a liquid suitably diffuses when the liquid is absorbed.

### Solution to Problem

The present inventor has found that suppressing of swelling in a vertical direction of water-absorbent resin particles when the water-absorbent resin particles absorb a liquid is effective in obtaining an absorbent article in which a liquid suitably diffuses in a plane direction of an absorber when the liquid is absorbed.

An aspect of the present invention provides water-absorbent resin particles as set out in the appended claims.

According to the above-mentioned water-absorbent resin particles, it is possible to obtain an absorbent article in which a liquid suitably diffuses in a plane direction of an absorber when the liquid is absorbed.

Another aspect of the present invention provides an absorber containing the above-mentioned water-absorbent resin particles.

Another aspect of the present invention provides a water-absorbent article including the above-mentioned absorber.

### Advantageous Effects of Invention

According to an aspect of the present invention which is set out in the appended claims, it is possible to provide water-absorbent resin particles and an absorber that provide an absorbent article in which a liquid suitably diffuses in a plane direction of the absorber when the liquid is absorbed. In addition, according to another aspect of the present invention which is set out in the appended claims, it is possible to provide an absorbent article in which a liquid suitably diffuses in a plane direction of an absorber when the liquid is absorbed. According to another aspect of the present invention which is set out in the appended claims, it is possible to provide use of a resin particle, an absorber, and an absorbent article to the absorption of a liquid.

**Brief Description of Drawings**

Fig. 1 is a cross-sectional view showing an example of an absorbent article.
Fig. 2 is a schematic view showing a measurement device of a swelling height.
Fig. 3 is a schematic view showing a measurement device of a water absorption amount under a load of water-absorbent resin particles.

**Description of Embodiments**

Hereinafter, embodiments of the present invention will be described in detail. However, the present invention is not limited to the following embodiments but is defined by the appended set of claims.

In the present specification, "acrylic" and "methacryl" are collectively referred to as "(meth)acrylic". Similarly, "acrylate" and "methacrylate" are also referred to as "(meth)acrylate". In a numerical value range described in stages in the present specification, an upper limit value or a lower limit value of the numerical value range of a stage can be optionally combined with the upper limit value or the lower limit value of the numerical value range of another stage. In a numerical value range described in the present specification, the upper limit value or the lower limit value of the numerical value range may be replaced with the value shown in the examples. "Water-soluble" means that it exhibits a solubility in water of 5% by mass or more at 25°C. Materials exemplified in the present specification may be used alone, or may be used in combination of two or more. The content of each component in the composition means the total amount of a plurality of substances present in the composition in a case where the plurality of substances corresponding to each component are present in the composition, unless otherwise specified. "Physiological saline" refers to 0.9% by mass sodium chloride aqueous solution.

In the water-absorbent resin particles of the present embodiment, the swelling height (gel swelling height) measured by the following procedures (1) to (6) is 7.0 to 9.2 mm.

(1) A container having a recess having the bottom area of 50 cm$^2$ is disposed in a state where the recess is open in a vertical direction.
(2) 1.00 g of water-absorbent resin particles are disposed in the recess.
(3) A non-woven fabric is disposed on the water-absorbent resin particles in the recess.
(4) A weight having the mass of 90 g is disposed on the non-woven fabric.
(5) Physiological saline is supplied into the recess.
(6) A movement distance in a vertical direction of the weight when 300 seconds have passed from the start of swelling of the water-absorbent resin particles is measured as the swelling height.

According to the water-absorbent resin particles of the present embodiment, it is possible to obtain an absorbent article in which a liquid suitably diffuses in a plane direction (a direction perpendicular to a thickness direction, a direction parallel to a liquid-absorbing surface) of an absorber when the liquid is absorbed. According to the water-absorbent resin particles of the present embodiment, it is possible to obtain an absorbent article which has suitable water-absorbent characteristics (water retention amount, water absorption rate, water absorption amount under a load) and in which a liquid suitably diffuses in a plane direction of an absorber when the liquid is absorbed.

In a case where the water-absorbent resin particles swell due to absorption of a liquid, the absorber and the absorbent article can swell in a thickness direction (a direction perpendicular to the liquid-absorbing surface). In a case of excessive swelling in a thickness direction, a user, a guardian thereof, a caregiver may misunderstand that a liquid has been sufficiently absorbed and it has been reached a state where further continuous use is not possible. In addition, in a wearing article such as a diaper, leakage can be suppressed by disposing a liquid absorbing member (for example, gather) in a plane direction of an absorber from the liquid absorbing position, but in the related art, sufficient measures have not been taken to swelling in a thickness direction. On the other hand, according to the water-absorbent resin particles of the present embodiment, it is possible to obtain an absorbent article in which a liquid suitably diffuses in a plane direction of an absorber when the liquid is absorbed while suppressing swelling of the absorber and the absorbent article in a thickness direction.

The swelling height is preferably 9.0 mm or less, 8.8 mm or less, or 8.6 mm or less from a viewpoint of easily obtaining an absorbent article in which a liquid suitably diffuses. The swelling height may be 8.5 mm or less, or 8.4 mm or less. The swelling height may be 8.0 mm or more. From these viewpoints, the swelling height may be 8.0 to 9.2 mm, or 8.0 to 9.0 mm.

In step (1) in a swelling height test, the container has a bottomed recess, and the container is disposed so that an opening direction of the recess is positioned in a vertical direction. The container has a bottom surface that is a flat surface, for example. In the container, a side wall forming the recess extends in the opening direction of the recess, for example. In a cross section perpendicular to the opening direction of the recess, the recess has a circular shape, for example. As the recess having a circular cross section, for example, a recess having the inner diameter of 80 mm and the bottom area of 50.24 cm$^2$ can be used.

In step (2), the water-absorbent resin particles can be disposed on the bottom surface of the recess. In step (2), the water-absorbent resin particles can be uniformly disposed on the bottom surface of the recess.

As the non-woven fabric in step (3), a liquid permeable non-woven fabric having the basis weight of 12 g/m$^2$ can be used. In step (3), the non-woven fabric can be brought into contact with the water-absorbent resin particles in the recess of the container. A size of the non-woven fabric is not particularly limited, and it is sufficient as long as a weight of step (4) and the water-absorbent resin particles do not come into direct contact with each other by using the non-woven fabric.

The weight in step (4) can move in a vertical direction without resistance in the inside of the container and may have a flat surface. The weight may be perforated with a plurality of holes through which the liquid can pass. The hole may extend in a direction substantially perpendicular to the flat surface of the weight (for example, a vertical direction), may extend by being inclined with respect to the flat surface of the weight, or may extend by bending. In step (4), the flat surface of the weight can be brought into contact with the non-woven fabric. In step (4), for example, the weight is gently placed on the non-woven fabric so that a load other than the load due to the mass of the weight is not applied to the water-absorbent resin particles. The weight may be a single member, or may be constituted of a plurality of members. The weight has a flat plate portion having a flat surface and a convex portion extending from the flat plate portion, for example.

The use amount of physiological saline in step (5) can be adjusted so that the water-absorbent resin particles are sufficiently immersed in physiological saline. The use amount of physiological saline is 20 to 200 g, for example.

As the start of swelling of the water-absorbent resin particles in step (6), the time when the water-absorbent resin particles swell and the weight starts to move can be used. By using the movement distance when 300 seconds have passed from the start of swelling, it is possible to easily select the water-absorbent resin particles that provide an absorbent article in which a liquid suitably diffuses.

The water-absorbent resin particles of the present embodiment are as defined in the appended set of claims. The water-absorbent resin particles of the present embodiment are better in absorbency of a body fluid such as urine, sweat, blood (for example, menstrual blood). The water-absorbent resin particles of the present embodiment can be used as a constituent component of the absorber of the present embodiment.

The water retention amount of physiological saline of the water-absorbent resin particles of the present embodiment is 30 to 80 g/g. The water retention amount is 34 g/g or more, 35 g/g or more, 40 g/g or more, 45 g/g or more, or 50 g/g or more from a viewpoint of easily increasing the absorption capacity of the absorbent article. The water retention amount is preferably 75 g/g or less, 70 g/g or less, 65 g/g or less, 60 g/g or less, or 55 g/g or less from a viewpoint of easily suppressing excessive swelling in the absorbent article. From these viewpoints, the water retention amount is preferably 30 to 70 g/g, 30 to 65 g/g, 30 to 55 g/g, 34 to 65 g/g, 34 to 60 g/g, or 34 to 55 g/g. As the water retention amount, the water retention amount at 25°C can be used. The water retention amount can be measured by the method described in examples to be described later.

The water absorption amount of physiological saline under the load of the water-absorbent resin particles of the present embodiment is preferably in the following range. The water absorption amount is preferably 10 mL/g or more, 12 mL/g or more, 15 mL/g or more, 18 mL/g or more, 20 mL/g or more, 24 mL/g or more, or 28 mL/g or more, from a viewpoint of easily obtaining an absorbent article in which a liquid suitably diffuses. The water absorption amount is preferably 40 mL/g or less, 35 mL/g or less, or 30 mL/g or less from a viewpoint of easily suppressing excessive swelling in the absorbent article. From these viewpoints, the water absorption amount is preferably 10 to 40 mL/g, 15 to 40 mL/g, 18 to 40 mL/g, 18 to 35 mL/g, 18 to 30 mL/g, 20 to 30 mL/g, or 24 to 30mL/g. As the water absorption amount of physiological saline under the load, the water absorption amount (25°C) at the load of 4.14 kPa can be used. The water absorption amount can be measured by the method described in examples to be described later.

The water absorption rate of physiological saline of the water-absorbent resin particles of the present embodiment is 60 second or less. The water absorption rate is preferably 57 seconds or less, or 55 seconds or less from a viewpoint of the liquid being suitably absorbed in the absorbent article. The water absorption rate is preferably 20 seconds or more, 25 seconds or more, 30 seconds or more, 33 seconds or more, 35 seconds or more, 40 seconds or more, or 45 seconds or more, from a viewpoint of easily preventing gel blocking caused by the liquid staying in a narrow portion. From these viewpoints, the water absorption rate is preferably 20 to 60 seconds, 30 to 60 seconds, 35 to 60 seconds, 40 to 60 seconds, 40 to 55 seconds, or 45 to 55 seconds. As the water absorption rate, the water absorption rate at 25°C can be used. The water absorption rate can be measured according to Vortex method (Japanese Industrial Standard JIS K 7224 (1996)). Specifically, it is possible to obtain the water absorption rate as the time [second] from after the addition of the water-absorbent resin particles until the vortex disappears and the liquid surface becomes flat, when 2.0 $\pm$ 0.002 g of the water-absorbent resin particles are added to 50 $\pm$ 0.1 g of physiological saline stirred at 600 rpm (rpm = min$^{-1}$).

Examples of the shape of the water-absorbent resin particles of the present embodiment include substantially spherical, crushed, and granular shapes. The medium particle diameter of the water-absorbent resin particles of the present embodiment is 300 to 700 $\mu$m and may be 300 to 600 $\mu$m, 340 to 600 $\mu$m, or 300 to 500 $\mu$m. The water-absorbent resin particles of the present embodiment may have a desired particle size distribution at the time of being obtained by a production method to be described later, but the particle size distribution may be adjusted by performing an operation such as particle size adjustment using classification with a sieve.

The water-absorbent resin particles of the present embodiment contain a crosslinking polymer having a structural unit derived from an ethylenically unsaturated monomer obtained by polymerizing a monomer containing an ethylenically unsaturated monomer, as polymer particles. That is, the water-absorbent resin particles of the present embodiment has a structural unit derived from an ethylenically unsaturated monomer. As the ethylenically unsaturated monomer, a water-soluble ethylenically unsaturated monomer can be used wherein the ethylenically unsaturated monomer comprises at least one compound selected from the group consisting of (meth)acrylic acid and a salt thereof. Examples of the polymerization method include a reverse phase suspension polymerization method, an aqueous solution polymerization method, a bulk polymerization method, and a precipitation polymerization method. Among these, the reverse phase suspension polymerization method or the aqueous solution polymerization method is preferable from a viewpoint of ensuring good water-absorbent characteristics of the obtained water-absorbent resin particles and facilitating control of the polymerization reaction. In the following, as a method for polymerizing an ethylenically unsaturated monomer, a reverse phase suspension polymerization method will be described as an example.

The ethylenically unsaturated monomer is preferably water-soluble, and examples thereof include (meth)acrylic acid and a salt thereof, 2-(meth)acrylamide-2-methylpropanesulfonic acid and a salt thereof, (meth)acrylamide, N, N-dimethyl (meth)acrylamide, 2-hydroxyethyl (meth)acrylate, N-methylol (meth)acrylamide, polyethylene glycol mono(meth)acrylate, N, N-diethylaminoethyl (meth)acrylate, N, N-diethylaminopropyl (meth)acrylate, and diethylaminopropyl (meth)acrylamide. In a case where the ethylenically unsaturated monomer has an amino group, the amino group may be quaternized. The ethylenically unsaturated monomer may be used alone, or may be used in combination of two or more, wherein in any embodiment of the invention the ethylenically unsaturated monomer comprises at least one compound selected from the group consisting of (meth)acrylic acid and a salt thereof. Functional groups such as a carboxyl group and an amino group of the above-mentioned monomers can function as functional groups capable of crosslinking in a surface crosslinking step to be described later.

Among these, from a viewpoint of industrial availability, the ethylenically unsaturated monomer comprises at least one compound selected from the group consisting of (meth)acrylic acid and a salt thereof, and preferably contains at least one compound selected from the group consisting of (meth)acrylic acid and a salt thereof, acrylamide, methacrylamide, and N, N-dimethylacrylamide, and more preferably contains at least one compound selected from the group consisting of (meth)acrylic acid and a salt thereof, and acrylamide. From a viewpoint of further enhancing the water-absorbent characteristics (water retention amount) in all embodiments of the invention the ethylenically unsaturated monomer contains at least one compound selected from the group consisting of (meth)acrylic acid and a salt thereof. That is, the water-absorbent resin particles have a structural unit derived from at least one selected from the group consisting of (meth)acrylic acid and a salt thereof.

As the monomer for obtaining the water-absorbent resin particles, a monomer other than the above-mentioned ethylenically unsaturated monomer may be used. Such a monomer can be used by being mixed with an aqueous solution containing the above-mentioned ethylenically unsaturated monomer, for example. The use amount of the ethylenically unsaturated monomer is 70 to 100 mol% with respect to a total amount of the monomer (the total amount of the monomer for obtaining the water-absorbent resin particles. For example, a total amount of the monomers that provide a structural unit of the crosslinking polymer. The same applies hereinafter). Among these, the ratio of (meth)acrylic acid and a salt thereof is 70 to 100 mol% with respect to the total amount of the monomers. "Ratio of (meth)acrylic acid and a salt thereof" means the ratio of the total amount of (meth)acrylic acid and a salt thereof.

According to the present embodiment, as an example of the water-absorbent resin particles, it is possible to provide the water-absorbent resin particles containing a crosslinking polymer having a structural unit derived from an ethylenically unsaturated monomer, in which the ethylenically unsaturated monomer contains at least one compound selected from the group consisting of (meth)acrylic acid and a salt thereof, and the ratio of (meth)acrylic acid and a salt thereof is 70 to 100 mol% with respect to the total amount of the monomer for obtaining the water-absorbent resin particles (for example, the total amount of the monomer that provides a structural unit of the crosslinking polymer), and these water-absorbent resin particles may have an aspect that the water retention amount of physiological saline is 30 to 80 g/g, the medium particle diameter is 300 to 700 μm, and the swelling height measured by the above-mentioned procedures of (1) to (6) is 7.0 to 9.2 mm and which further complies with the features of the water-absorbent resin particles set out in the appended set of claims.

The ethylenically unsaturated monomer is usually preferably used as an aqueous solution. The concentration of the ethylenically unsaturated monomer in the aqueous solution containing the ethylenically unsaturated monomer (hereinafter, simply referred to as "monomer aqueous solution") is preferably 20% by mass or more and a saturated concentration or less, more preferably 25 to 70% by mass, and further more preferably 30 to 55% by mass. Examples of the water used in the aqueous solution include tap water, distilled water, and ion-exchanged water.

In a case where the ethylenically unsaturated monomer has an acid group, the monomer aqueous solution may be used by neutralizing the acid group with an alkaline neutralizing agent. The degree of neutralization of the ethylenically unsaturated monomer by the alkaline neutralizing agent is preferably 10 to 100 mol%, more preferably 50 to 90 mol%, and further more preferably 60 to 80 mol% of the acid group in the ethylenically unsaturated monomer, from a viewpoint of increasing an osmotic pressure of the obtained water-absorbent resin particles, and further increasing the water-absorbent characteristics (water retention amount). Examples of the alkaline neutralizing agent include alkali metal salts such as sodium hydroxide, sodium carbonate, sodium hydrogen carbonate, potassium hydroxide, and potassium carbonate; and ammonia. The alkaline neutralizing agent may be used alone, or may be used in combination of two or more. The alkaline neutralizing agent may be used in the form of an aqueous solution to simplify the neutralization operation. Neutralization of the acid group of the ethylenically unsaturated monomer can be performed by adding an aqueous solution of sodium hydroxide, potassium hydroxide, dropwise in the above-mentioned monomer aqueous solution and mixing therewith.

In a reverse phase suspension polymerization method, a monomer aqueous solution is dispersed in a hydrocarbon dispersion medium in the presence of a surfactant, and polymerization of the ethylenically unsaturated monomer can be performed using a radical polymerization initiator. As the radical polymerization initiator, a water-soluble radical polymerization initiator can be used.

Examples of the surfactant include a nonionic surfactant, and an anionic surfactant. Examples of the nonionic surfactant include sorbitan fatty acid ester, (poly)glycerin fatty acid ester ("(poly)" means both of a case where there is a prefix of "poly" and a case where there is no prefix thereof. The same applies hereinafter), sucrose fatty acid ester, polyoxyethylene sorbitan fatty acid ester, polyoxyethylene glycerin fatty acid ester, sorbitol fatty acid ester, polyoxyethylene sorbitol fatty acid ester, polyoxyethylene alkyl ether, polyoxyethylene alkyl phenyl ether, polyoxyethylene castor oil, polyoxyethylene hydrogenated castor oil, alkylallyl formaldehyde condensed polyoxyethylene ether, polyoxyethylene polyoxypropylene block copolymer, polyoxyethylene polyoxypropyl alkyl ether, and polyethylene glycol fatty acid ester. Examples of the anionic surfactant include fatty acid salt, alkylbenzene sulfonate, alkylmethyl taurate, polyoxyethylene alkylphenyl ether sulfate, polyoxyethylene alkyl ether sulfonate, phosphate ester of polyoxyethylene alkyl ether, and phosphate ester of polyoxyethylene alkylallyl ether. The surfactant may be used alone, or may be used in combination of two or more.

From a viewpoint of a good state of the W/O type reverse phase suspension, easily obtaining water-absorbent resin particles having a suitable particle diameter, and industrial availability, the surfactant preferably contains at least one compound selected from the group consisting of a sorbitan fatty acid ester, a polyglycerin fatty acid ester, and a sucrose fatty acid ester. From a viewpoint of easily obtaining an appropriate particle size distribution of the water-absorbent resin particles, and from a viewpoint of easily improving the water-absorbent characteristics of the water-absorbent resin particles and the performance of the absorbent article using the same, the surfactant preferably contains sucrose fatty acid ester, and more preferably contains sucrose stearic acid ester.

The use amount of the surfactant is preferably 0.05 to 10 parts by mass, more preferably 0.08 to 5 parts by mass, and further more preferably 0.1 to 3 parts by mass with respect to 100 parts by mass of the monomer aqueous solution from a viewpoint of obtaining a sufficient effect on the use amount and economic efficiency.

In the reverse phase suspension polymerization, a polymeric dispersant may be used in combination with the above-mentioned surfactant. Examples of the polymeric dispersant include maleic anhydride-modified polyethylene, maleic anhydride-modified polypropylene, maleic anhydride-modified ethylene/propylene copolymer, maleic anhydride-modified EPDM (ethylene propylene diene terpolymer), maleic anhydride-modified polybutadiene, maleic anhydride/ethylene copolymer, maleic anhydride/propylene copolymer, maleic anhydride/ethylene/propylene copolymer, maleic anhydride/butadiene copolymer, polyethylene, polypropylene, ethylene/propylene copolymer, oxidized polyethylene, oxidized polypropylene, oxidized ethylene/propylene copolymer, ethylene/acrylic acid copolymer, ethyl cellulose, and ethyl hydroxyethyl cellulose. The polymeric dispersant may be used alone or may be used in combination of two or more. From a viewpoint of better dispersion stability of the monomer, the polymeric dispersant is preferably at least one selected from the group consisting of maleic anhydride-modified polyethylene, maleic anhydride-modified polypropylene, maleic anhydride-modified ethylene/propylene copolymer, maleic anhydride/ethylene copolymer, maleic anhydride/propylene copolymer, maleic anhydride/ethylene/propylene copolymer, polyethylene, polypropylene, ethylene/propylene copolymer, oxidized polyethylene, oxidized polypropylene, and oxidized ethylene/propylene copolymer.

The use amount of the polymeric dispersant is preferably 0.05 to 10 parts by mass, more preferably 0.08 to 5 parts by mass, and further more preferably 0.1 to 3 parts by mass with respect to 100 parts by mass of the monomer aqueous solution, from a viewpoint of obtaining a sufficient effect on the use amount and economic efficiency.

The hydrocarbon dispersion medium may contain at least one compound selected from the group consisting of chain aliphatic hydrocarbons having 6 to 8 carbon atoms and alicyclic hydrocarbons having 6 to 8 carbon atoms. Examples of the hydrocarbon dispersion medium include chain aliphatic hydrocarbons such as n-hexane, n-heptane, 2-methylhexane, 3-methylhexane, 2,3-dimethylpentane, 3-ethylpentane, and n-octane; alicyclic hydrocarbons such as cyclohexane, methylcyclohexane, cyclopentane, methylcyclopentane, trans-1,2-dimethylcyclopentane, cis-1,3-dimethylcyclopentane, and trans-1,3-dimethylcyclopentane; and aromatic hydrocarbons such as benzene, toluene, and xylene. The hydrocarbon dispersion medium may be used alone, or may be used in combination of two or more.

The hydrocarbon dispersion medium may contain at least one selected from the group consisting of n-heptane and cyclohexane from a viewpoint of industrial availability and stable quality. In addition, from the same viewpoint, as the mixture of the above-mentioned hydrocarbon dispersion medium, for example, commercially available Exxsol Heptane (manufactured by ExxonMobil: containing 75% to 85% of n-heptane and isomeric hydrocarbons) may be used.

The use amount of the hydrocarbon dispersion medium is preferably 30 to 1000 parts by mass, more preferably 40 to 500 parts by mass, and further more preferably 50 to 400 parts by mass with respect to 100 parts by mass of the monomer aqueous solution, from a viewpoint of appropriately removing the heat of polymerization and easily controlling the polymerization temperature. In a case where the use amount of the hydrocarbon dispersion medium is 30 parts by mass or more, the polymerization temperature tends to be easily controlled. In a case where the use amount of the hydrocarbon dispersion medium is 1000 parts by mass or less, the productivity of polymerization tends to be improved, which is economical.

The radical polymerization initiator is preferably water-soluble, and examples thereof include persulfates such as potassium persulfate, ammonium persulfate, and sodium persulfate; peroxides such as methyl ethyl ketone peroxide, methyl isobutyl ketone peroxide, di-t-butyl peroxide, t-butyl cumylperoxide, t-butylperoxyacetate, t-butylperoxyisobutyrate, t-butylperoxypivalate, and hydrogen peroxide; azo compounds such as 2,2'-azobis(2-amidinopropane) dihydrochloride, 2,2'-azobis [2-(N-phenylamidino) propane] dihydrochloride, 2,2'-azobis [2-(N-allylamidino) propane] dihydrochloride, 2,2'-azobis [2-(2-imidazoline-2-yl) propane] dihydrochloride, 2,2'-azobis {2-[1-(2-hydroxyethyl) -2-imidazoline-2-yl] propane} dihydrochloride, 2,2'-azobis {2-methyl-N-[1,1-bis (hydroxymethyl)-2-hydroxyethyl] propionamide}, 2,2'-azobis [2-methyl-N-(2-hydroxyethyl)-propionamide], and 4,4'-azobis (4-cyanovaleric acid). The radical polymerization initiator may be used alone, or may be used in combination of two or more. The radical polymerization initiator is preferably at least one selected from the group consisting of potassium persulfate, ammonium persulfate, sodium persulfate, 2,2'-azobis (2-amidinopropane) dihydrochloride, 2,2'-azobis [2- (2-imidazoline-2-yl) propane] dihydrochloride, and 2,2'-azobis {2-[1-(2-hydroxyethyl)-2-imidazoline-2-yl] propane} dihydrochloride.

The use amount of the radical polymerization initiator may be 0.05 to 10 mmol with respect to 1 mol of the ethylenically unsaturated monomer. In a case where the use amount of the radical polymerization initiator is 0.05 mmol or more, the polymerization reaction does not require a long time and is efficient. In a case where the use amount of the radical polymerization initiator is 10 mmol or less, the occurrence of a rapid polymerization reaction is easily inhibited.

The above-mentioned radical polymerization initiator can also be used as a redox polymerization initiator in combination with a reducing agent such as sodium sulfite, sodium hydrogen sulfite, ferrous sulfate, and L-ascorbic acid.

At the time of the polymerization reaction, the monomer aqueous solution used for the polymerization may contain a chain transfer agent. Examples of the chain transfer agent include hypophosphites, thiols, thiolic acids, secondary alcohols, and amines.

The monomer aqueous solution used for the polymerization may contain a thickener in order to control the particle diameter of the water-absorbent resin particles. Examples of the thickener include hydroxyethyl cellulose, hydroxypropyl cellulose, methyl cellulose, carboxymethyl cellulose, polyethylene glycol, polyacrylamide, polyethyleneimine, dextrin, sodium alginate, polyvinyl alcohol, polyvinylpyrrolidone, and polyethylene oxide. In a case where the stirring speed at the time of polymerization is the same, the higher the viscosity of the monomer aqueous solution, the larger the medium particle diameter of the obtained particles tends to be.

Crosslinking by self-crosslinking may occur during polymerization, but according to the invention crosslinking is performed by using an internal crosslinking agent. When an internal crosslinking agent is used, the water-absorbent characteristics (swelling height, water retention amount) of the water-absorbent resin particles are easily controlled. The internal crosslinking agent is added to a reaction solution during the polymerization reaction. Examples of the internal crosslinking agent include di or tri (meth)acrylic acid esters of polyols such as ethylene glycol, propylene glycol, trimethylolpropane, glycerin, polyoxyethylene glycol, polyoxypropylene glycol, and polyglycerin; unsaturated polyesters obtained by reacting the above-mentioned polyols with unsaturated acids (such as maleic acid and fumaric acid); bis (meth)acrylamides such as N, N'-methylene bis (meth)acrylamide; di or tri (meth)acrylic acid esters obtained by reacting polyepoxide with (meth)acrylic acid; di (meth)acrylic acid carbamil esters obtained by reacting polyisocyanate (such as tolylene diisocyanate and hexamethylene diisocyanate) with hydroxyethyl (meth)acrylate; compounds having two or more polymerizable unsaturated groups such as allylated starch, allylated cellulose, diallyl phthalate, N, N', N"-triallyl isocyanurate, and divinylbenzene; polyglycidyl compounds such as (poly)ethylene glycol diglycidyl ether, (poly) propylene glycol diglycidyl ether, (poly)glycerin diglycidyl ether, (poly)glycerin triglycidyl ether, (poly)propylene glycol polyglycidyl ether, and polyglycerol polyglycidyl ether; haloepoxy compounds such as epichlorohydrin, epibromhydrin, and $\alpha$-methylepichlorohydrin; and compounds having two or more reactive functional groups such as isocyanate compounds (2,4-tolylene diisocyanate and hexamethylene diisocyanate). The internal crosslinking agent may be used alone, or may be used in combination of two or more. The internal crosslinking agent is preferably a polyglycidyl compound, more preferably a diglycidyl ether compound, and further more preferably at least one selected from the group consisting of (poly)ethylene glycol diglycidyl ether, (poly)propylene glycol diglycidyl ether, and (poly)glycerin diglycidyl ether.

The use amount of the internal crosslinking agent is preferably 30 mmol or less, more preferably 0.01 to 10 mmol, further more preferably 0.012 to 5 mmol, particularly preferably 0.015 to 1 mmol, extremely preferably 0.02 to 0.1 mmol, and extraordinarily preferably 0.02 to 0.05 mmol per 1 mol of the ethylenically unsaturated monomer, from a viewpoint of easily obtaining an absorbent article in which a liquid suitably diffuses, and from a viewpoint of suppressing water-soluble property by appropriately crosslinking the obtained polymer to easily obtain the sufficient water absorption amount.

It is possible to perform heating while stirring in a state of mixing an ethylenically unsaturated monomer, a radical polymerization initiator, a surfactant, a polymeric dispersant, a hydrocarbon dispersion medium, (if necessary, additionally an internal crosslinking agent), and to perform reverse phase suspension polymerization in a water-in-oil system.

When performing the reverse phase suspension polymerization, a monomer aqueous solution containing an ethylenically unsaturated monomer is dispersed in a hydrocarbon dispersion medium in the presence of a surfactant (if necessary, additionally a polymeric dispersant). At this time, before the start of the polymerization reaction, the timing of adding the surfactant, the polymeric dispersant, may be either before or after the addition of the monomer aqueous solution.

Among these, from a viewpoint of easily reducing the amount of the hydrocarbon dispersion medium remaining in the obtained water-absorbent resin, it is preferable to perform polymerization after dispersing the monomer aqueous solution in the hydrocarbon dispersion medium in which the polymeric dispersant is dispersed and then further dispersing the surfactant.

Reverse phase suspension polymerization can be performed in one stage, or in multiple stages of two or more stages. Reverse phase suspension polymerization is preferably performed in two to three stages from a viewpoint of increasing productivity.

In a case where reverse phase suspension polymerization is performed in multiple stages of two or more stages, a first stage reverse phase suspension polymerization is performed, an ethylenically unsaturated monomer is added to the reaction mixture obtained in the first polymerization reaction and mixed therewith, and second and subsequent stages of reverse phase suspension polymerization may be performed in the same method as the first stage. In the reverse phase suspension polymerization in each stage of the second and subsequent stages, in addition to the ethylenically unsaturated monomer, the above-mentioned radical polymerization initiator and/or internal crosslinking agent is preferably added in a range of a molar ratio of each component with respect to the above-mentioned ethylenically unsaturated monomer, based on an amount of the ethylenically unsaturated monomer added at the time of the second and subsequent stages of reverse phase suspension polymerization, to perform reverse phase suspension polymerization. In the reverse phase suspension polymerization in each stage of second and subsequent stages, an internal crosslinking agent may be used if necessary. The internal crosslinking agent is preferably added within a range of the molar ratio of each component with respect to the above-mentioned ethylenically unsaturated monomer based on the amount of the ethylenically unsaturated monomer provided in each stage, to perform reverse phase suspension polymerization.

The temperature of the polymerization reaction varies depending on the used radical polymerization initiator, and the temperature is preferably 20°C to 150°C, and more preferably 40°C to 120°C, from a viewpoint of rapidly proceeding the polymerization and shortening the polymerization time to enhance economic efficiency, and easily removing polymerization heat and smoothly performing reaction. The reaction time is usually 0.5 to 4 hours. The completion of the polymerization reaction can be confirmed by stopping the temperature rise in the reaction system. Thus, the polymer of the ethylenically unsaturated monomer is usually obtained in a state of a hydrogel.

After the polymerization, a post-polymerization crosslinking agent may be added to the obtained hydrogel-like polymer and heated to perform crosslinking. By performing post-polymerization crosslinking, a degree of crosslinking of the hydrogel-like polymer can be increased, and the water-absorbent characteristics (swelling height, water retention amount) can be further improved.

Examples of the post-polymerization crosslinking agent include polyols such as ethylene glycol, propylene glycol, 1,4-butanediol, trimethylolpropane, glycerin, polyoxyethylene glycol, polyoxypropylene glycol, and polyglycerin;

compounds having two or more epoxy groups such as (poly)ethylene glycol diglycidyl ether, (poly)propylene glycol diglycidyl ether, and (poly)glycerin diglycidyl ether; haloepoxy compounds such as epichlorohydrin, epibromhydrin, and α-methylepichlorohydrin; compounds having two or more isocyanate groups such as 2,4-tolylene diisocyanate and hexamethylene diisocyanate; oxazoline compounds such as 1,2-ethylenebisoxazoline; carbonate compounds such as ethylene carbonate; and hydroxyalkylamide compounds such as bis [N, N-di (β-hydroxyethyl)] adipamide. Among these, polyglycidyl compounds such as (poly)ethylene glycol diglycidyl ether, (poly)glycerin diglycidyl ether, (poly)glycerin triglycidyl ether, (poly)propylene glycol polyglycidyl ether, and polyglycerol polyglycidyl ether are preferable. The crosslinking agent may be used alone, or may be used in combination of two or more.

The amount of the post-polymerization crosslinking agent is preferably 30 mmol or less, more preferably 10 mmol or less, further more preferably 0.01 to 5 mmol, particularly preferably 0.012 to 1 mmol, extremely preferably 0.015 to 0.1 mmol, and extraordinarily preferably 0.02 to 0.05 mmol per 1 mol of the ethylenically unsaturated monomer, from a viewpoint of easily obtaining suitable water-absorbent characteristics (swelling height, water retention amount) by appropriately crosslinking the obtained hydrogel-like polymer.

The timing of adding the post-polymerization crosslinking agent may be after the polymerization of the ethylenically unsaturated monomer used for the polymerization, and in the case of multiple-stage polymerization, it is preferable to add after the multiple-stage polymerization. Considering fluctuation in water due to heat generation at the time of polymerization and after polymerization, retention due to process delay, opening of the system at the time of addition of the crosslinking agent, addition of water due to the addition of the crosslinking agent, the post-polymerization crosslinking agent is preferably added in a region of [water content (immediately after polymerization) ± 3% by mass] from a viewpoint of water content (to be described later).

Subsequently, the polymer particles (for example, polymer particles having a structural unit derived from an ethylenically unsaturated monomer) are obtained by drying in order to remove water from the obtained hydrogel-like polymer. Examples of a drying method include (a) a method of removing water by performing azeotropic distillation by heating from outside in a state where a hydrogel-like polymer is dispersed in a hydrocarbon dispersion medium, and refluxing the hydrocarbon dispersion medium, (b) a method of taking out a hydrogel-like polymer by decantation and drying under reduced pressure, and (c) a method of filtering the hydrogel-like polymer with a filter and drying under reduced pressure. Among these, it is preferable to use the method (a) due to the simplicity in the production process.

It is possible to adjust the particle diameter of water-absorbent resin particles by adjusting a rotation speed of a stirrer during the polymerization reaction, or by adding a flocculant into the system after the polymerization reaction or in the initial stage of drying. By adding a flocculant, it is possible to increase the particle diameter of the obtained water-absorbent resin particles. As the flocculant, an inorganic flocculant can be used. Examples of the inorganic flocculant (for example, powdered inorganic flocculant) include silica, zeolite, bentonite, aluminum oxide, talc, titanium dioxide, kaolin, clay, and hydrotalcite. From a viewpoint of better flocculation effect, the flocculant is preferably at least one selected from the group consisting of silica, aluminum oxide, talc, and kaolin.

In the reverse phase suspension polymerization, a method of adding the flocculant is preferably a method of preliminarily dispersing a flocculant in a hydrocarbon dispersion medium or water of the same type as that used in the polymerization, and then mixing into a hydrocarbon dispersion medium containing a hydrogel-like polymer under stirring.

The addition amount of the flocculant is preferably 0.001 to 1 part by mass, more preferably 0.005 to 0.5 part by mass, and further more preferably 0.01 to 0.2 parts by mass with respect to 100 parts by mass of the ethylenically unsaturated monomer used for the polymerization. In a case where the addition amount of the flocculant is within the above-mentioned range, water-absorbent resin particles having a target particle size distribution can be easily obtained.

In the production of the water-absorbent resin particles, it is preferable to perform surface crosslinking of a surface portion (surface and in the vicinity of surface) of a hydrogel-like polymer using a surface crosslinking agent in a drying step (water removing step) or any subsequent steps. By performing surface crosslinking, the water-absorbent characteristics (swelling height, water retention amount, ) of the water-absorbent resin particles is easily controlled.

The surface crosslinking is preferably performed at the timing when the hydrogel-like polymer has a specific water content. The timing of surface crosslinking is preferably when the water content of the hydrogel-like polymer is 5% to 50% by mass, more preferably when the water content of the hydrogel-like polymer is 10% to 40% by mass, and further more preferably when the water content of the hydrogel-like polymer is 15% to 35% by mass. The water content (mass%) of the hydrogel-like polymer is calculated by the following formula.

$$\text{Water content} = [Ww/(Ww + Ws)] \times 100$$

Ww: water amount of a hydrogel-like polymer obtained by adding water amount used if necessary when mixing a flocculant, a surface crosslinking agent, to an amount obtained by subtracting water amount discharged to the outside of the system in the drying step, from water amount contained in a monomer aqueous solution before polymerization in the entire polymerization step.

Ws: Solid content calculated from the charged amount of materials such as ethylenically unsaturated monomer, crosslinking agent, and initiator that constitute a hydrogel-like polymer.

Examples of the surface crosslinking agent include compounds having two or more reactive functional groups. Examples of the surface crosslinking agent include polyols such as ethylene glycol, propylene glycol, 1,4-butanediol, trimethylolpropane, glycerin, polyoxyethylene glycol, polyoxypropylene glycol, and polyglycerin; polyglycidyl compounds such as (poly)ethylene glycol diglycidyl ether, (poly)glycerin diglycidyl ether, (poly)glycerin triglycidyl ether, trimethylpropane triglycidyl ether (poly)propylene glycol polyglycidyl ether, and (poly)glycerol polyglycidyl ether; haloepoxy compounds such as epichlorohydrin, epibromhydrin, and $\alpha$-methylepichlorohydrin; isocyanate compounds such as 2,4-tolylene diisocyanate and hexamethylenediisocyanate; oxetane compounds such as 3-methyl-3-oxetane methanol, 3-ethyl-3-oxetane methanol, 3-butyl-3-oxetane methanol, 3-methyl-3-oxetane ethanol, 3-ethyl-3-oxetane ethanol, and 3-butyl-3-oxetane ethanol; oxazoline compounds such as 1,2-ethylenebisoxazoline; carbonate compounds such as ethylene carbonate; and hydroxyalkylamide compounds such as bis [N, N-di ($\beta$-hydroxyethyl)] adipamide. The surface crosslinking agent may be used alone, or may be used in combination of two or more. The surface crosslinking agent is preferably a polyglycidyl compound, and more preferably at least one selected from the group consisting of (poly)ethylene glycol diglycidyl ether, (poly)glycerin diglycidyl ether, (poly)glycerin triglycidyl ether, (poly)propylene glycol polyglycidyl ether, and polyglycerol polyglycidyl ether.

The use amount of the surface crosslinking agent is preferably 0.01 to 20 mmol, more preferably 0.05 to 10 mmol, further more preferably 0.1 to 5 mmol, particularly preferably 0.15 to 1 mmol, and extremely preferably 0.2 to 0.5 mmol per 1 mol of the ethylenically unsaturated monomer used for polymerization, from a viewpoint of easily obtaining suitable water-absorbent characteristics (swelling height, water retention amount,).

After surface crosslinking, it is possible to obtain polymer particles which are surface-crosslinked dried products by distilling water and a hydrocarbon dispersion medium with a known method.

According to the invention the polymer particles contained in the water-absorbent resin particles can be obtained by using an internal crosslinking agent used at the time of polymerizing the monomer and an external crosslinking agent (a post-polymerization crosslinking agent used after the polymerization of the monomer, and a surface crosslinking agent used in the drying step after polymerization of a monomer or subsequent steps) used after polymerization of the monomer. The ratio of the use amount of the external crosslinking agent with respect to the internal crosslinking agent (external crosslinking agent/internal crosslinking agent) is 5 to 100, preferably 10 to 80, further more preferably 15 to 50, and particularly preferably 15 to 30, from a viewpoint of easily obtaining suitable water-absorbent characteristics (swelling height, water retention amount,). The water-absorbent resin particles contain polymer particles which are reaction products using an internal crosslinking agent and an external crosslinking agent. The ratio of the use amount of the external crosslinking agent to the internal crosslinking agent in the polymer particles is in the above range.

In addition to the polymer particles, the water-absorbent resin particles of the present embodiment can further contain additional components such as a gel stabilizer, a metal chelating agent (ethylenediaminetetraacetic acid and a salt thereof, diethylenetriamine pentaacetate and a salt thereof, for example, diethylenetriamine pentasodium pentaacetate), and a flowability improver (lubricant). Additional components can be disposed inside the polymer particles, on the surface of the polymer particles, or both thereof.

The water-absorbent resin particles may contain a plurality of inorganic particles disposed on the surface of the polymer particles. For example, by mixing the polymer particles and the inorganic particles, it is possible to dispose the inorganic particles on the surface of the polymer particles. The inorganic particles may be silica particles such as amorphous silica.

In a case where the water-absorbent resin particles include inorganic particles disposed on the surface of the polymer particles, the content of the inorganic particles may be in the following range based on the total mass of the polymer particles. The content of the inorganic particles may be 0.05% by mass or more, 0.1% by mass or more, 0.15% by mass or more, or 0.2% by mass or more. The content of the inorganic particles may be 5.0% by mass or less, 3.0% by mass or less, 1.0% by mass or less, 0.5% by mass or less, or 0.3% by mass or less.

The inorganic particles here usually have a minute size as compared with the size of the polymer particles. For example, the average particle diameter of the inorganic particles may be 0.1 to 50 $\mu$m, 0.5 to 30 $\mu$m, or 1 to 20 $\mu$m. The average particle diameter can be measured by a pore electric resistance method or a laser diffraction/scattering method depending on the characteristics of the particles.

The absorber of the present embodiment contains the water-absorbent resin particles of the present embodiment. The absorber of the present embodiment may contain a fibrous substance, for example, is a mixture containing water-absorbent resin particles and the fibrous substance. For example, the structure of the absorber may be a structure in which the water-absorbent resin particles and the fibrous substance are uniformly mixed, may be a structure in which the water-absorbent resin particles are sandwiched between the fibrous substances formed in the form of a sheet or a layer, or may be other structures.

Examples of the fibrous substance include finely pulverized wood pulp; cotton; cotton linter; rayon; cellulosic fibers such as cellulose acetate; synthetic fibers such as polyamide, polyester and polyolefin; and a mixture of these fibers. The fibrous substance may be used alone, or may be used in combination of two or more. As the fibrous substance, hydrophilic fibers can be used.

In order to enhance the morphological retention before and during use of the absorber, the fibers may be adhered to each other by adding an adhesive binder to the fibrous substance. Examples of the adhesive binder include thermal bonding synthetic fibers, hot melt adhesives, and adhesive emulsions. The adhesive binder may be used alone, or may be used in combination of two or more.

Examples of the thermal bonding synthetic fiber include a total fusion type binder such as polyethylene, polypropylene, and an ethylene-propylene copolymer; and a non-total fusion type binder made of a side-by-side or core-sheath structure of polypropylene and polyethylene. In the above-mentioned non-total fusion type binder, only the polyethylene portion can be thermal-bonded.

Examples of the hot melt adhesive include a mixture of a base polymer such as ethylene-vinyl acetate copolymer, styrene-isoprene-styrene block copolymer, styrene-butadiene-styrene block copolymer, styrene-ethylene-butylene-styrene block copolymer, styrene-ethylene-propylene-styrene block copolymer, and amorphous polypropylene with a tackifier, a plasticizer, an antioxidant.

Examples of the adhesive emulsion include a polymerization product of at least one monomer selected from the group consisting of methyl methacrylate, styrene, acrylonitrile, 2-ethylhexyl acrylate, butyl acrylate, butadiene, ethylene, and vinyl acetate.

The absorber of the present embodiment may contain an inorganic powder (for example, amorphous silica), a deodorant, an antibacterial agent, a dye, a pigment, a fragrance, a sticking agent In a case where the water-absorbent resin particles contain inorganic particles, the absorber may contain an inorganic powder in addition to the inorganic particles of the water-absorbent resin particles.

The shape of the absorber of the present embodiment may be a sheet shape, for example. The thickness of the absorber (for example, thickness of the sheet shaped absorber) may be 0.1 to 20 mm or 0.3 to 15 mm.

The content of the water-absorbent resin particles in the absorber may be 2% to 100% by mass, 10% to 80% by mass, or 20% to 60% by mass with respect to the total of the water-absorbent resin particles and the fibrous substance, from a viewpoint of easily obtaining sufficient water absorption performance.

The content of the water-absorbent resin particles in the absorber is preferably 100 to 1000 g, more preferably 150 to 800 g, and further more preferably 200 to 700 g per 1 $m^2$ of the absorber from a viewpoint of easily obtaining sufficient water absorption performance. The content of the fibrous substance in the absorber is preferably 50 to 800 g, more preferably 100 to 600 g, and further more preferably 150 to 500 g per 1 $m^2$ of the absorber from a viewpoint of easily obtaining sufficient water absorption performance.

The absorbent article of the present embodiment includes an absorber of the present embodiment. Examples of the absorbent article of the present embodiment include a core wrap that retains an absorber and prevents falloff or flow of a constituent member of the absorber; a liquid permeable sheet disposed on the outermost part at the side where the liquid to be absorbed enters; and a liquid impermeable sheet disposed on the outermost part at the opposite side to the side where the liquid to be absorbed enters. Examples of the absorbent article include diapers (for example, paper diapers), toilet training pants, incontinence pads, sanitary materials (sanitary napkins, tampons), sweat pads, pet sheets, portal toilet members, and animal excrement treatment materials.

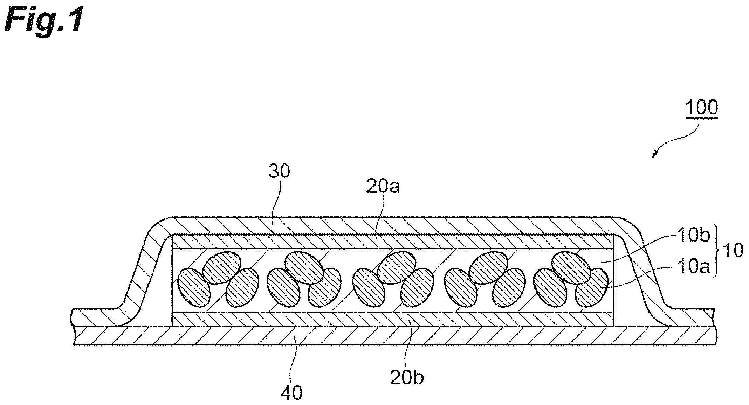

Fig. 1 is a cross-sectional view showing an example of an absorbent article. An absorbent article 100 shown in Fig. 1 includes an absorber 10, core wraps 20a and 20b, a liquid permeable sheet 30, and a liquid impermeable sheet 40. In the absorbent article 100, the liquid impermeable sheet 40, the core wrap 20b, the absorber 10, the core wrap 20a, and the liquid permeable sheet 30 are laminated in this order. In Fig. 1, there is a portion shown so that there is a gap between the members, but the members may be in close contact with each other without the gap.

The absorber 10 has a water-absorbent resin particle 10a of the present embodiment and a fiber layer 10b containing a fibrous substance. The water-absorbent resin particles 10a are dispersed in the fiber layer 10b.

The core wrap 20a is disposed on one surface side of the absorber 10 (upper side of the absorber 10 in Fig. 1) in a state of being in contact with the absorber 10. The core wrap 20b is disposed on the other surface side of the absorber 10 (lower side of the absorber 10 in Fig. 1) in a state of being in contact with the absorber 10. The absorber 10 is disposed between the core wrap 20a and the core wrap 20b. Examples of the core wraps 20a and 20b include tissues, non-woven fabrics, woven fabrics, synthetic resin films having liquid permeation holes, and net-like sheets having a mesh. The core wrap 20a and the core wrap 20b have a main surface having the same size as that of the absorber 10, for example.

The liquid permeable sheet 30 is disposed on the outermost part at the side where the liquid to be absorbed enters. The liquid permeable sheet 30 is disposed on the core wrap 20a in a state of being in contact with the core wrap 20a. Examples of the liquid permeable sheet 30 include a non-woven fabric made of a synthetic resin such as polyethylene, polypropylene, polyester, and polyamide, and a porous sheet. The liquid impermeable sheet 40 is disposed on the outermost part at the opposite side to the liquid permeable sheet 30 in the absorbent article 100. The liquid impermeable sheet 40 is disposed on a lower side of the core wrap 20b in a state of being in contact with the core wrap 20b. Examples of the liquid impermeable sheet 40 include a sheet made of a synthetic resin such as polyethylene, polypropylene, and polyvinyl chloride, and a sheet made of a composite material of these synthetic resins and a non-woven fabric. The liquid permeable sheet 30 and the liquid impermeable sheet 40 have a main surface wider than the main surface of the absorber 10, and outer edges of the liquid permeable sheet 30 and the liquid impermeable sheet 40 are present around the absorber 10 and the core wraps 20a and 20b.

The magnitude relationship between the absorber 10, the core wraps 20a and 20b, the liquid permeable sheet 30, and the liquid impermeable sheet 40 is not particularly limited, and is appropriately adjusted according to the use of the absorbent article. In addition, the method of retaining the shape of the absorber 10 using the core wraps 20a and 20b is not particularly limited, and as shown in Fig. 1, the absorber may be wrapped by a plurality of core wraps, and the absorber is wrapped by one core wrap.

The absorber may be adhered to a top sheet. In a case where the absorber is sandwiched or covered by the core wrap, it is preferable that at least the core wrap and the top sheet are adhered to each other, and it is more preferable that the core wrap and the top sheet are adhered to each other and the core wrap and the absorber are adhered to each other. Examples of a method of adhering the absorber include a method of adhering by applying a hot melt adhesive to the top sheet at predetermined intervals in a striped shape, a spiral shape, in a width direction; and a method of adhering using a water-soluble binder such as starch, carboxymethyl cellulose, polyvinyl alcohol, polyvinylpyrrolidone, and other water-soluble polymers. In addition, in a case where the absorber contains thermal bonding synthetic fibers, a method of adhering by thermal bonding of the thermal bonding synthetic fibers may be adopted.

According to the present embodiment, it is possible to provide a liquid absorbing method using the water-absorbent resin particles, the absorber or the absorbent article of the present embodiment. The liquid absorbing method of the present embodiment includes a step of bringing the liquid to be absorbed into contact with the water-absorbent resin particles, the absorber or the absorbent article of the present embodiment.

According to the present embodiment, it is possible to provide a method for improving a diffusion distance of a liquid in a plane direction of an absorber in an absorbent article, which is a method for improving a diffusion distance using the water-absorbent resin particles, the absorber or the absorbent article of the present embodiment. According to the present embodiment, it is possible to provide a method for suppressing the swelling of the water-absorbent resin particles in a vertical direction by using the water-absorbent resin particles having the above-mentioned swelling height of 10 mm or less. According to the present embodiment, it is possible to provide a method for producing water-absorbent resin particles, including a selection step of selecting water-absorbent resin particles based on the above-mentioned swelling height. In the selection step, for example, the water-absorbent resin particles are selected based on whether or not the above-mentioned swelling height is 10 mm or less.

## Examples

Hereinafter, contents of the present invention will be described in further detail using examples and comparative examples, but the present invention is not limited to the following examples.

<Preparation of water-absorbent resin particles>

(Example 1)

A round-bottomed cylindrical separable flask with the inner diameter of 11 cm and the internal volume of 2 L equipped with a reflux cooling device, a dropping funnel, a nitrogen gas introduction tube, and a stirrer (a stirrer blade having two stages of four inclined paddle blades with the blade diameter of 5 cm) was prepared. Into this flask, 293 g of n-heptane was added as a hydrocarbon dispersion medium and 0.736 g of a maleic anhydride-modified ethylene/propylene copolymer (manufactured by Mitsui Chemicals, Inc., High Wax 1105A) was added as a polymeric dispersant to obtain a mixture. The dispersant was dissolved by raising the temperature to 80°C while stirring the mixture, and then the mixture was cooled to 50°C.

Subsequently, 92.0 g of 80.5% by mass aqueous acrylic acid solution (acrylic acid: 1.03 mol) was added into a beaker having the internal volume of 300 mL as a water-soluble ethylenically unsaturated monomer. Subsequently, while cooling from the outside, 147.7 g of 20.9% by mass sodium hydroxide aqueous solution was added dropwise into the beaker to perform 75 mol% of neutralization on acrylic acid. Thereafter, 0.092 g of hydroxyethyl cellulose (manufactured by Sumitomo Seika Chemicals Co., Ltd., HEC AW-15F) as a thickener, 0.092 g (0.339 mmol) of 2,2'-azobis (2-amidinopropane) dihydrochloride and 0.018 g (0.068 mmol) of potassium persulfate as a water-soluble radical polymerization initiator, and 0.005 g (0.029 mmol) of ethylene glycol diglycidyl ether as an internal crosslinking agent were added and then dissolved therein to prepare a first stage aqueous solution.

Then, the first stage aqueous solution was added into the above-mentioned separable flask while stirring at the rotation speed of 550 rpm of the stirrer, and then stirring was performed for 10 minutes. Thereafter, a surfactant solution obtained by heat-dissolving 0.736 g of sucrose stearic acid ester (surfactant, manufactured by Mitsubishi-Chemical Foods Corporation, Ryoto Sugar Ester S-370, HLB value: 3) in 6.62 g of n-heptane was added into the separable flask. Then, the inside of the system was sufficiently replaced with nitrogen while stirring at the stirring speed of 550 rpm of the stirrer. Thereafter, the flask was immersed in a water bath at 70°C to raise the temperature, and polymerization was performed for 60 minutes to obtain a first stage polymerization slurry solution.

Subsequently, 128.8 g of 80.5% by mass aqueous acrylic acid solution (acrylic acid: 1.43 mol) was added into another beaker having the internal volume of 500 mL as a water-soluble ethylenically unsaturated monomer. Subsequently, while cooling from the outside, 159.0 g of 27% by mass sodium hydroxide aqueous solution was added dropwise into the beaker to perform 75 mol% of neutralization on acrylic acid. Thereafter, 0.129 g (0.475 mmol) of 2,2'-azobis (2-amidinopropane) dihydrochloride and 0.026 g (0.095 mmol) of potassium persulfate as water-soluble radical polymerization initiators were added and then dissolved therein to prepare a second stage aqueous solution.

Subsequently, while stirring at the rotation speed of 1000 rpm of the stirrer, the inside of the above-mentioned separable flask was cooled to 25°C, and then the total amount of the above-mentioned second stage aqueous solution was added to the above-mentioned first stage polymerization slurry solution. Subsequently, after replacing the inside of the system with nitrogen for 30 minutes, the flask was immersed in a water bath at 70°C again to raise the temperature, and the polymerization reaction was performed for 60 minutes. Thereafter, 0.580 g of 2% by mass ethylene glycol diglycidyl ether aqueous solution (ethylene glycol diglycidyl ether: 0.067 mmol) was added as a post-polymerization crosslinking agent to obtain a second stage hydrogel-like polymer.

To the above-mentioned second stage hydrogel-like polymer, 0.265 g of 45% by mass diethylenetriamine pentasodium pentaacetate aqueous solution was added under stirring. Thereafter, the flask was immersed in an oil bath set at 125°C, and 223.7 g of water was extracted to the outside of the system while refluxing n-heptane by azeotropic distillation of n-heptane and water. Then, 4.42 g of 2% by mass ethylene glycol diglycidyl ether aqueous solution (ethylene glycol diglycidyl ether: 0.507 mmol) was added into the flask as a surface crosslinking agent, and then the mixture was held at 83°C for 2 hours.

Thereafter, n-heptane was evaporated at 125°C and dried to obtain polymer particles (dried product). After passing these polymer particles through a sieve having the opening of 850 $\mu$m, 0.2% by mass of amorphous silica (Tokusil NP-S manufactured by Oriental Silicas Corporation) was mixed with the polymer particles based on the total mass of the polymer particles to obtain 229.6 g of water-absorbent resin particles containing amorphous silica. The medium particle diameter of the water-absorbent resin particles was 346 $\mu$m. In Example 1, the ratio of the use amount of the external crosslinking agent with respect to the use amount of the internal crosslinking agent was 19.8 in terms of molar ratio.

(Example 2)

230.6 g of water-absorbent resin particles were obtained in the same manner as that in Example 1, except that, in the second stage hydrogel-like polymer, 231.7 g of water was extracted to the outside of the system by azeotropic distillation. The medium particle diameter of the water-absorbent resin particles was 361 $\mu$m.

(Example 3)

231.1 g of water-absorbent resin particles were obtained in the same manner as that in Example 1, except that, in the second stage hydrogel-like polymer, 234.2 g of water was extracted to the outside of the system by azeotropic distillation. The medium particle diameter of the water-absorbent resin particles was 355 $\mu$m.

(Comparative Example 1)

231.0 g of water-absorbent resin particles were obtained in the same manner as that in Example 1, except that, in the preparation of the first stage aqueous solution, 0.0736 g (0.272 mmol) of potassium persulfate was used as a water-soluble radical polymerization initiator, 2,2'-azobis (2-amidinopropane) dihydrochloride was not added, and 0.010 g (0.057 mmol) of ethylene glycol diglycidyl ether was used as an internal crosslinking agent; in the preparation of the second stage aqueous solution, 0.090 g (0.334 mmol) of persulfate was used as a water-soluble radical polymerization initiator, 2,2'-azobis (2-amidinopropane) dihydrochloride was not added, and 0.012 g (0.069 mmol) of ethylene glycol diglycidyl ether was used as an internal crosslinking agent; in the preparation of the hydrogel-like polymer, after performing polymerization reaction for 60 minutes, 0.265 g of 45% by mass diethylenetriamine pentasodium pentaacetate aqueous solution was added without adding the crosslinking agent; in the hydrogel-like polymer after the second stage polymerization, 247.9 g of water was extracted to the outside of the system by azeotropic distillation; and 0.5% by mass amorphous silica with respect to the mass of the polymer particles was mixed with the polymer particles. The medium particle diameter of the water-absorbent resin particles was 355 $\mu$m. In Comparative Example 1, the ratio of the use amount of the external crosslinking agent with respect to the use amount of the internal crosslinking agent was 4.0 in terms of molar ratio.

(Comparative Example 2)

230.8 g of water-absorbent resin particles were obtained in the same manner as that in Comparative Example 1, except that, in the hydrogel-like polymer after the second stage polymerization, 256.1 g of water was extracted to the outside of the system by azeotropic distillation, and 0.1% by mass amorphous silica with respect to the mass of the polymer particles was mixed with the polymer particles. The medium particle diameter of the water-absorbent resin particles was 349 μm.

(Comparative Example 3)

230.8 g of water-absorbent resin particles were obtained in the same manner as that in Comparative Example 1, except that, in the hydrogel-like polymer after the second stage polymerization, 278.9 g of water was extracted to the outside of the system by azeotropic distillation, and 0.2% by mass amorphous silica with respect to the mass of the polymer particles was mixed with the polymer particles. The medium particle diameter of the water-absorbent resin particles was 347 μm.

(Comparative Example 4)

A round-bottomed cylindrical separable flask having 4 side wall baffles (baffle width: 7 mm), with the inner diameter of 11 cm and the internal volume of 2 L, equipped with a reflux cooling device, a dropping funnel, a nitrogen gas introduction tube, and a stirrer (a stirrer blade having two stages of four inclined paddle blades with the blade diameter of 5 cm (those surface-treated with fluororesin)) was prepared. Into this flask, 451.4 g of n-heptane as a hydrocarbon dispersion medium was put, and 0.984 g of sorbitan monolaurate (Nonion LP-20R, HLB value: 8.6, manufactured by NOF CORPORATION) was added as a surfactant to obtain a mixture. The sorbitan monolaurate was dissolved in n-heptane by raising the temperature to 50°C while stirring this mixture at the rotation speed of 300 rpm of the stirrer, and then the internal temperature was cooled to 40°C.

Subsequently, 92.0 g of 80.5% by mass aqueous acrylic acid solution (acrylic acid: 1.03 mol) was added into a triangular flask having the internal volume of 500 mL. Subsequently, while cooling with ice from the outside, 147.7 g of 20.9% by mass sodium hydroxide aqueous solution was added dropwise into the triangular flask to perform 75 mol% of neutralization. Thereafter, 0.101 g (0.374 mmol) of potassium persulfate as a radical polymerization initiator was added to an acrylic acid partial neutralization aqueous solution, and then dissolved therein to prepare a monomer aqueous solution.

After adding the above-mentioned monomer aqueous solution into the above-mentioned separable flask, the inside of the system was sufficiently replaced with nitrogen. Thereafter, while stirring at the rotation speed of 700 rpm of the stirrer, the flask was immersed in a water bath at 70°C and then held for 60 minutes to obtain a hydrogel-like polymer.

Subsequently, while stirring at the rotation speed of 1000 rpm of the stirrer, a mixture obtained by previously dispersing 0.092 g of amorphous silica (powdered inorganic flocculant, manufactured by Oriental Silicas Corporation, Tokusil NP-S) to 100 g of n-heptane was added to a polymer solution containing the above-mentioned hydrogel-like polymer, n-heptane, and a surfactant, and then mixing was performed for 10 minutes to obtain a mixture solution. Thereafter, the flask containing the reaction solution was immersed in an oil bath at 125°C, and 112 g of water was extracted to the outside of the system while refluxing n-heptane by azeotropic distillation of n-heptane and water. Thereafter, 4.97 g of 10% by mass ethylene glycol diglycidyl ether (ethylene glycol diglycidyl ether: 2.85 mmol) was added as a post-polymerization crosslinking agent, and then the internal temperature was held at 80 ± 2°C for 2 hours.

Thereafter, water and n-heptane were evaporated, and dried until almost no evaporated product from the system was left out. Subsequently, the flask was once removed from the oil bath, and 13.8 g of water was sprayed at the flow rate of 0.3 g per second. Thereafter, polymer particles (dried product) were obtained by holding the system at 80°C for 30 minutes while blowing nitrogen into the system at the flow rate of 200 mL per minute. The polymer particles were passed through a sieve having the opening of 850 μm to obtain 90.5 g of water-absorbent resin particles. The medium particle diameter of the water-absorbent resin particles was 356 μm.

(Comparative Example 5)

A round-bottomed cylindrical separable flask having 4 side wall baffles (baffle width: 7 mm), with the inner diameter of 11 cm and the internal volume of 2 L, equipped with a reflux cooling device, a dropping funnel, a nitrogen gas introduction tube, and a stirrer (a stirrer blade having two stages of four inclined paddle blades with the blade diameter of 5 cm (those surface-treated with fluororesin)) was prepared. Into this flask, 451 g of n-heptane as a hydrocarbon dispersion medium was put, and 0.984 g of sorbitan monolaurate (Nonion LP-20R, HLB value: 8.6, manufactured by NOF CORPORATION) was added as a surfactant to obtain a mixture. The sorbitan monolaurate was dissolved in n-heptane by raising the temperature to 50°C while stirring this mixture at the rotation speed of 300 rpm of the stirrer, and then the internal temperature was cooled to 40°C.

Subsequently, 92.0 g of 80.5% by mass aqueous acrylic acid solution (acrylic acid: 1.03 mol) was added into a triangular flask having the internal volume of 500 mL. Subsequently, while cooling with ice from the outside, 147.7 g of 20.9% by mass sodium hydroxide aqueous solution was added dropwise into the triangular flask to perform 75 mol% of neutralization. Thereafter, 0.101 g (0.374 mmol) of potassium persulfate as a radical polymerization initiator was added to an acrylic acid partial neutralization aqueous solution, and then dissolved therein to prepare a monomer aqueous solution.

After adding the above-mentioned monomer aqueous solution into the above-mentioned separable flask, the inside of the system was sufficiently replaced with nitrogen. Thereafter, while stirring at the rotation speed of 700 rpm of the stirrer, the flask was immersed in a water bath at 70°C, and then held for 60 minutes to obtain a reaction solution.

Thereafter, the flask containing the reaction solution was immersed in an oil bath at 125°C, and 129 g of water was extracted to the outside of the system while refluxing n-heptane by azeotropic distillation of n-heptane and water. Thereafter, 4.14 g of 2% by mass ethylene glycol diglycidyl ether aqueous solution (ethylene glycol diglycidyl ether: 0.48 mmol) was added as a surface crosslinking agent, and then the internal temperature was held at $80 \pm 2$°C for 2 hours.

Thereafter, n-heptane was evaporated at 125°C and dried to obtain polymer particles (dried product). The polymer particles were passed through a sieve having the opening of 850 $\mu$m to obtain 90.0 g of water-absorbent resin particles. The medium particle diameter of the water-absorbent resin particles was 180 $\mu$m.

<Measurement of medium particle diameter>

The above-mentioned medium particle diameter of the water-absorbent resin particles was measured by the following procedure. Specifically, JIS standard sieves were combined in the following order from the top: a sieve having the opening of 600 $\mu$m, a sieve having the opening of 500 $\mu$m, a sieve having the opening of 425 $\mu$m, a sieve having the opening of 300 $\mu$m, a sieve having the opening of 250 $\mu$m, a sieve having the opening of 180 $\mu$m, a sieve having the opening of 150 $\mu$m, and a tray. 50 g of the water-absorbent resin particles were put in the topmost sieve among the combined sieves, and classification was performed by shaking for 10 minutes using a Ro-tap shaker. After classification, the mass of the particles remaining on each sieve was calculated as a mass percentage with respect to the total amount, and the particle size distribution was obtained. The relationship between the opening of the sieve and the integrated value of the mass percentage of the particles remaining on the sieve was plotted on logarithmic probability paper by integrating in the order from the one having the largest particle diameter on the sieve with respect to this particle size distribution. By connecting the plots on the probability paper with a straight line, the particle diameter corresponding to the cumulative mass percentage of 50% by mass was obtained as the medium particle diameter.

<Measurement of swelling height>

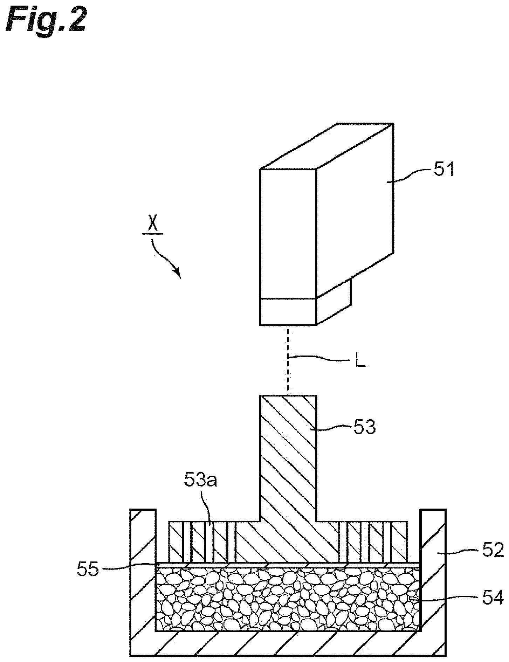

The swelling height was measured using a measurement device X shown in Fig. 2. The measurement device X shown in Fig. 2 is constituted of a movement distance measurement device 51, a recess circular cup (height 45 mm, outer diameter 90 mm, recess depth 40 mm, recess inner diameter 80 mm) 52, and a plastic convex circular piston (outer diameter 79 mm) 53. The convex circular piston 53 has a flat plate portion having a flat surface and a convex portion (cross-sectional shape: circular, diameter: 15 mm, cross-sectional area: 176.7 mm$^2$) extending from the center of the flat surface of the flat plate portion. In the flat plate portion of the convex circular piston 53, sixty through holes 53a having the diameter of 2 mm penetrating the flat plate portion are uniformly formed. The through hole 53a extending to a lower portion of the convex portion in a vertical direction extends by being inclined from a position around the convex portion on the flat surface of the flat plate portion with respect to the flat surface, and another through hole 53a not extending to the lower portion of the convex portion in a vertical direction extends in a vertical direction with respect to the flat surface of the flat plate portion (in Fig. 2, for convenience of illustration, all the through holes 53a are shown so as to extend in a vertical direction). The measurement device X has a sensor (lower portion of the movement distance measurement device 51, manufactured by KEYENCE CORPORATION, IL-100) capable of measuring the displacement of the distance by a laser beam L in a unit of 0.01 mm. In a state where an analyzer and the measurement device X are connected to each other via an amplifier unit (IL-1000, manufactured by KEYENCE CORPORATION), a channel unit (NR-TH08, manufactured by KEYENCE CORPORATION), and a data logger (NR-500, manufactured by KEYENCE CORPORATION), the displacement of the distance can be measured using a data analysis software (WAVE LOGGER, manufactured by KEYENCE CORPORATION). A predetermined amount of water-absorbent resin particles 54 can be uniformly sprayed in the recess circular cup 52. A non-woven fabric 55 (liquid permeable non-woven fabric having the basis weight of 12 g/m$^2$) can be disposed on the water-absorbent resin particles 54 in the recess of the recess circular cup 52. The convex circular piston 53 can uniformly apply the load of 90 g to the water-absorbent resin particles 54 via the non-woven fabric 55.

The measurement was performed in an environment of the temperature of 25°C and the humidity of 60 $\pm$ 10%. 1.0 g of the water-absorbent resin particles 54 were uniformly scattered on an entire surface of the bottom in the recess of the recess circular cup 52, and then the non-woven fabric 55 was disposed on the water-absorbent resin particles 54. Subsequently, after the convex circular piston 53 was gently placed on the non-woven fabric 55, the laser beam L of the sensor of the movement distance measurement device 51 was adjusted so as to be positioned at a central portion of the tip of the convex portion of the convex circular piston 53. In a state where 90 g of load was applied to the water-absorbent resin particles 54 by the convex circular piston 53, physiological saline (20 g) adjusted to 25°C in advance was passed through the through hole 53a of the convex circular piston 53 and injected into the recess circular cup 52, a timing when it was sensed that the water-absorbent resin particles 54 swelled and pushed up the convex circular piston 53 (automatic measurement started when the displacement of 0.5 mm was observed) was denoted as water absorption start (0 second), and a distance at which the water-absorbent resin particles 54 swelled and the convex circular piston 53 was pushed up (displacement difference of the convex circular piston 53 in the direction perpendicular to the bottom surface of the recess of the recess circular cup 52) was measured. A water level of the physiological saline was checked every 3 seconds from the start of the injection of physiological saline, and the injection of physiological saline was intermittently continued so as to maintain the water surface in the vicinity a height of the flat surface of the flat plate portion of the convex circular piston 53. The movement distance of the convex circular piston 53 after 5 minutes (300 seconds) from the start of water absorption was obtained as a swelling height [mm].

<Water retention amount of water-absorbent resin particles>

The water retention amount (room temperature, 25°C $\pm$ 2°C) of physiological saline of the water-absorbent resin particles was measured by the following procedure. First, a cotton bag (Membroroad No. 60, width 100 mm $\times$ length 200 mm) containing 2.0 g of the weighted water-absorbent resin particles was placed in a beaker having the internal volume of 500 mL. After pouring 500 g of 0.9% by mass sodium chloride aqueous solution (physiological saline) into a cotton bag containing the water-absorbent resin particles at one time so that lumps cannot be formed, an upper portion of the cotton bag was tied with a rubber band and the cotton bag was left for 30 minutes to swell the water-absorbent resin particles. The cotton bag after 30 minutes was dehydrated for 1 minute using a dehydrator (product number: H-122, manufactured by KOKUSAN Co., Ltd.) set to have the centrifugal force of 167 G, and then the mass Wa [g] of the cotton bag containing swollen gel after dehydration was measured. The same operation was performed without adding the water-absorbent resin particles, the empty mass Wb [g] at the time when the cotton bag was wet was measured, and the water retention amount of physiological saline of the water-absorbent resin particles was calculated from the formula below. The results are shown in Table 1.

$$\text{Water retention amount [g/g]} = (Wa - Wb)/2.0$$

<Water absorption rate of water-absorbent resin particles>

The water absorption rate of physiological saline of the water-absorbent resin particles was measured by the following procedure based on the Vortex method. First, 50 $\pm$ 0.1 g of 0.9% by mass sodium chloride aqueous solution (physiological saline) adjusted to the temperature of 25 $\pm$ 0.2°C in a constant temperature water tank was weighed in a beaker having the internal volume of 100 mL. Subsequently, a vortex was generated by stirring at the rotation speed of 600 rpm using a magnetic stirrer bar (8 mm$\varphi$ $\times$ 30 mm, without ring). 2.0 $\pm$ 0.002 g of the water-absorbent resin particles were added to the sodium chloride aqueous solution at one time. The time [seconds] from after the addition of the water-absorbent resin particles until the vortex on the liquid surface converged was measured, and this time was obtained as the water absorption rate of the water-absorbent resin particles. The results are shown in Table 1.

<Water retention amount under load of water-absorbent resin particles>

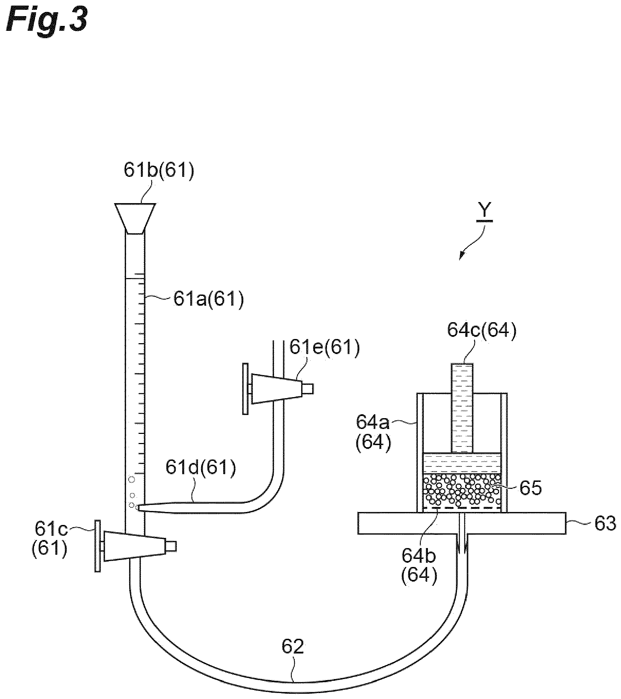

A water retention amount of physiological saline under a load (under pressure) of the water-absorbent resin particles (room temperature, 25°C $\pm$ 2°C) was measured using a measurement device Y shown in Fig. 3. The measurement device Y is constituted of a burette unit 61, a conduit 62, a measurement table 63, and a measurement unit 64 placed on the measurement table 63. The burette unit 61 has a burette 61a extending in a vertical direction, a rubber stopper 61b disposed at the upper end of the burette 61a, a cock 61c disposed at the lower end of the burette 61a, an air introduction tube 61d of which one end extends into the burette 61a in the vicinity of the cock 61c, and a cock 61e disposed on the other end side of the air introduction tube 61d. The conduit 62 is attached between the burette unit 61 and the measurement table 63. The inner diameter of the conduit 62 is 6 mm. At the central portion of the measurement table 63, a hole having the diameter of 2 mm is formed and the conduit 62 is connected. The measurement unit 64 has a cylinder 64a (made of acrylic resin (plexiglass)), a nylon mesh 64b adhered to the bottom of the cylinder 64a, and a weight 64c. The inner diameter of the cylinder 64a is 20 mm. The opening of the nylon mesh 64b is 75 μm (200 mesh). Then, at the time of measurement, the water-absorbent resin particles 65 to be measured are uniformly scattered on the nylon mesh 64b. The weight 64c has the diameter of 19 mm and the mass of the weight 64c is 120 g. The weight 64c is placed on the water-absorbent resin particles 65, and can apply the load of 4.14 kPa to the water-absorbent resin particles 65.

After putting 0.100 g of the water-absorbent resin particles 65 in the cylinder 64a of the measurement device Y, the weight 64c was placed and the measurement was started. Since the same volume of air as physiological saline absorbed by the water-absorbent resin particles 65 is quickly and smoothly supplied to the inside of the burette 61a from the air introduction tube, the amount of reduction in the water level of physiological saline inside the burette 61a corresponds to the amount of physiological saline absorbed by the water-absorbent resin particles 65. A scale of the burette 61a is engraved from top to bottom in increments of 0 mL to 0.5 mL; as a water level of physiological saline, a scale Va of the burette 61a before the start of water absorption and a scale Vb of the burette 61a after 60 minutes from the start of water absorption are read; and a water absorption amount under the load was calculated by the following formula. The results are shown in Table 1.

$$\text{Water absorption under pressure [mL / g]} = (Vb-Va)/0.1$$

<Evaluation of absorber performance>

(Preparation of test solution)

60 g of sodium chloride, 1.8 g of calcium chloride dihydrate, 3.6 g of magnesium chloride hexahydrate, and an appropriate amount of distilled water were put in a container having the internal volume of 10 L, and then completely dissolved. Subsequently, after adding 0.02 g of polyoxyethylene nonylphenyl ether, a total mass of the aqueous solution was adjusted to 6000 g by adding distilled water. Subsequently, a test solution (artificial urine) was obtained by coloring with a small amount of blue No. 1.

(Preparation of absorbent article)

Using an air flow type mixer (Padformer, manufactured by O-tec Co., Ltd.), 10 g of the water-absorbent resin particles and 10 g of pulverized pulp were uniformly mixed by air papermaking to prepare a sheet-shaped absorber having the size of 40 cm × 12 cm. Subsequently, the absorber was disposed on a core wrap (tissue paper) having the same size as that of the absorber and having the basis weight of 16 g/m$^2$, and then a core wrap (tissue paper) having the same size as that of the absorber and having the basis weight of 16 g/m$^2$ was disposed on an upper surface of the absorber. A laminate was obtained by applying the load of 196 kPa to the absorber sandwiched by the core wraps for 30 seconds. In addition, an air-through type porous liquid permeable sheet made of polyethylene-polypropylene having the same size as that of the absorber and having the basis weight of 22 g/m$^2$ was disposed on the upper surface of the laminate, and a liquid impermeable sheet made of polyethylene having the same size and the same basis weight was disposed on a lower surface of the laminate to prepare an absorbent article.

(Evaluation of diffusion length and permeation rate)

In a room having the temperature of 25 ± 2°C, a measurement instrument equipped with a liquid injection cylinder having an opening having the inner diameter of 3 cm was placed in a central portion of an absorbent article disposed on a horizontal table. Subsequently, 50 mL of the test solution adjusted to 25 ± 1°C was injected into the cylinder at one time (supplied from a vertical direction) and the stopwatch was started. An absorption time from the start of injection until the test solution was completely absorbed in the absorber was measured. This operation was performed twice more at intervals of 30 minutes (three times in total), and the total value of the absorption time was obtained as the permeation rate (unit: second). The shorter the permeation rate, the better it is. In addition, after 120 minutes from the start of the first injection of the test solution, a spreading dimension in a longitudinal direction (plane direction of the absorber in the absorbent article) of the absorbent article in which the test solution was permeated (a distance (diffusion distance) between both ends of the diffusion region of the test solution, passing through the central portion of the absorbent article. unit: cm) was measured. The diffusion length was measured in a unit of 0.5 cm. The results are shown in Table 1.

[Table 1]

| | Swelling height [mm] | Water retention amount [g/g] | Water absorption rate [sec] | Water absorption amount under load [mL/g] | Absorber performance | |
|---|---|---|---|---|---|---|
| | | | | | Permeation rate [sec] | Diffusion distance [cm] |
| Example 1 | 8.6 | 34 | 45 | 28 | 40 | 20.5 |
| Example 2 | 9.1 | 48 | 54 | 21 | 45 | 19.0 |
| Example 3 | 8.4 | 51 | 48 | 18 | 46 | 19.0 |
| Comparative Example 1 | 11.7 | 35 | 36 | 23 | 43 | 18.0 |
| Comparative Example 2 | 12.1 | 43 | 37 | 18 | 46 | 17.5 |
| Comparative Example 3 | 13.4 | 50 | 44 | 9 | 53 | 17.5 |
| Comparative Example 4 | 13.2 | 36 | 5 | 18 | 46 | 16.0 |
| Comparative Example 5 | 12.6 | 40 | 2 | 14 | 118 | 15.0 |

According to Table 1, it is confirmed that, by reducing a swelling height, an absorbent article in which a liquid suitably diffuses in a plane direction of an absorber when the liquid is absorbed is obtained.

**Reference Signs List**

10: absorber, 10a, 54, 65: water-absorbent resin particle, 10b: fiber layer, 20a, 20b: core wrap, 30: liquid permeable sheet, 40: liquid impermeable sheet, 51: movement distance measurement device, 52: recess circular cup, 53: convex circular piston, 53a: through-hole, 55: non-woven fabric, 61: burette unit, 61a: burette, 61b: rubber stopper, 61c: cock, 61d: air introduction tube, 61e: cock, 62: conduit, 63: measurement table, 64: measurement unit, 64a: cylinder, 64b: nylon mesh, 64c: weight, 100: absorbent article, L: laser beam, X, Y: Measurement device.

**Claims**

1.  Water-absorbent resin particles comprising:

a crosslinking polymer comprising a structural unit derived from an ethylenically unsaturated monomer, as polymer particles,
wherein the ethylenically unsaturated monomer comprises at least one compound selected from the group consisting of (meth)acrylic acid and a salt thereof, a ratio of the (meth)acrylic acid and the salt thereof is 70 to 100 mol% with respect to a total amount of monomers for obtaining the water-absorbent resin particles,
a water retention amount of physiological saline is 30 to 80 g/g when determined according to the method of the detailed description,
a water absorption rate of physiological saline is 60 seconds or less when determined according to the method of the detailed description,
a medium particle diameter is 300 to 700 $\mu$m wherein the medium particle diameter is determined by classification of the particles by sieving, calculating the mass percentage with respect to the total amount to obtain a particle size distribution and determining the cumulative mass percentage of 50% by mass, and
a swelling height measured by the following procedures (1) to (6) is 7.0 to 9.2 mm,

(1) a container having a recess having a bottom area of 50 cm$^2$ is disposed in a state where the recess is open in a vertical direction,

(2) 1.00 g of water-absorbent resin particles are disposed in the recess,
(3) a non-woven fabric is disposed on the water-absorbent resin particles in the recess,
(4) a weight having a mass of 90 g is disposed on the non-woven fabric,
(5) physiological saline is supplied into the recess,
(6) a movement distance in a vertical direction of the weight when 300 seconds have passed from the start of swelling of the water-absorbent resin particles is measured as the swelling height, wherein the polymer particles are obtainable by using an internal crosslinking agent at the time of polymerizing the monomer, and using an external crosslinking agent after polymerization of the monomer, wherein the ratio of the external crosslinking agent with respect to the internal crosslinking agent is 5 to 100.

2. An absorber comprising:
the water-absorbent resin particles according to claim 1.

3. An absorbent article comprising:
the absorber according to claim 2.

4. The absorbent article according to claim 3, which is a diaper.


**Patentansprüche**

1. Wasserabsorbierende Harzpartikel, die Folgendes umfassen:

ein vernetzendes Polymer, das eine von einem ethylenisch ungesättigten Monomer abgeleitete Struktureinheit als Polymerpartikel umfasst,
wobei das ethylenisch ungesättigte Monomer mindestens eine Verbindung umfasst, die aus der Gruppe ausgewählt ist, die aus (Meth)acrylsäure und einem Salz davon besteht, wobei ein Verhältnis der (Meth)acrylsäure und ihres Salzes 70 bis 100 Mol-% in Bezug auf eine Gesamtmenge von Monomeren zum Erhalten der wasserabsorbierenden Harzpartikel beträgt,
eine Wasserretentionsmenge der physiologischen Kochsalzlösung 30 bis 80 g/g beträgt, wenn sie gemäß dem Verfahren der ausführlichen Beschreibung bestimmt wird,
eine Wasserabsorptionsrate der physiologischen Kochsalzlösung 60 Sekunden oder weniger beträgt, wenn sie gemäß dem Verfahren der ausführlichen Beschreibung bestimmt wird,
ein mittlerer Partikeldurchmesser 300 bis 700 $\mu$m beträgt, wobei der mittlere Partikeldurchmesser durch Klassifizierung der Partikel durch Sieben, Berechnen des Massenprozentsatzes in Bezug auf die Gesamtmenge zum Erhalten einer Partikelgrößenverteilung und Bestimmen des kumulativen Massenprozentsatzes von 50 Massen-% bestimmt wird, und
eine durch die folgenden Verfahren (1) bis (6) gemessene Quellhöhe 7,0 bis 9,2 mm beträgt,

(1) ein Behälter mit einer Aussparung mit einer Bodenfläche von 50 cm$^2$ in einem Zustand angeordnet ist, in dem die Aussparung in senkrechter Richtung offen ist,
(2) 1,00 g wasserabsorbierender Harzpartikel in der Aussparung angeordnet sind,
(3) ein Vliesstoff auf den wasserabsorbierenden Harzpartikeln in der Aussparung angeordnet ist,
(4) ein Gewicht mit einer Masse von 90 g auf dem Vliesstoff angeordnet ist,
(5) physiologische Kochsalzlösung in die Aussparung zugeführt wird,
(6) eine Bewegungsdistanz in senkrechter Richtung des Gewichts nach Ablauf von 300 Sekunden seit Beginn des Quellens der wasserabsorbierenden Harzpartikel als Quellhöhe gemessen wird, wobei die Polymerpartikel unter Verwendung eines internen Vernetzungsmittels zum Zeitpunkt der Polymerisation des Monomers und unter Verwendung eines externen Vernetzungsmittels nach der Polymerisation des Monomers erhältlich sind, wobei das Verhältnis des externen Vernetzungsmittels in Bezug auf das interne Vernetzungsmittel 5 bis 100 beträgt.

2. Absorber, der Folgendes umfasst:
die wasserabsorbierenden Harzpartikel nach Anspruch 1.

3. Absorbierender Artikel, der Folgendes umfasst:
den Absorber nach Anspruch 2.

4. Absorbierender Artikel nach Anspruch 3, der eine Windel ist.

**Revendications**

1. Particules de résine absorbant l'eau comprenant :

   un polymère de réticulation comprenant une unité structurale dérivée d'un monomère éthyléniquement insaturé, comme des particules de polymère,
   où le monomère éthyléniquement insaturé comprend au moins un composé sélectionné parmi le groupe consistant en acide (méth)acrylique et un sel de celui-ci, un rapport de l'acide (méth)acrylique et le sel de celui-ci est de 70 à 100 mol% par rapport à une quantité totale de monomères pour obtenir les particules de résine absorbant l'eau,
   une quantité de rétention d'eau de sérum physiologique est de 30 à 80 g/g lorsqu'elle est déterminée selon le procédé de la description détaillée,
   un taux d'absorption d'eau de sérum physiologique est de 60 secondes ou moins lorsqu'il est déterminé selon le procédé de la description détaillée,
   un diamètre de particule moyen est de 300 à 700 $\mu$m, où le diamètre de particule moyen est déterminé par classification des particules en tamisant, en calculant le pourcentage en masse par rapport à la quantité totale pour obtenir une distribution par taille de particules et en déterminant le pourcentage en masse cumulée de 50 % en masse, et
   une hauteur de gonflement mesurée par les procédures suivantes (1) à (6) est de 7,0 à 9,2 mm,

   (1) un récipient ayant une cavité ayant une surface de fond de 50 cm$^2$ est disposé dans un état où la cavité est ouverte dans une direction verticale,
   (2) 1,00 g de particules de résine absorbant l'eau sont disposées dans la cavité,
   (3) un tissu non tissé est disposé sur les particules de résine absorbant l'eau dans la cavité,
   (4) un poids ayant une masse de 90 g est disposé sur le tissu non-tissé,
   (5) un sérum physiologique est introduit dans la cavité,
   (6) une distance de mouvement dans une direction verticale du poids lorsque 300 secondes se sont écoulées depuis le début du gonflement des particules de résine absorbant l'eau est mesurée comme la hauteur de gonflement,

   où les particules de polymère peuvent être obtenues en utilisant un agent de réticulation interne au moment de la polymérisation du monomère, et en utilisant un agent de réticulation externe après la polymérisation du monomère, où le rapport de l'agent de réticulation externe par rapport à l'agent de réticulation interne est de 5 à 100.

2. Absorbeur comprenant :
   les particules de résine absorbant l'eau selon la revendication 1.

3. Article absorbant comprenant :
   l'absorbeur selon la revendication 2.

4. Article absorbant selon la revendication 3, qui est une couche.

Fig.1

# Fig.2

*Fig.3*

**EP 3 896 120 B1**

## REFERENCES CITED IN THE DESCRIPTION

**Patent documents cited in the description**

- JP H6345819 A
- JP H9510889 A
- WO 2017170605 A1